# EUROPEAN PATENT SPECIFICATION

(11) **EP 1 923 003 B1**
(45) Date of publication and mention of the grant of the patent: **11.07.2018**
(21) Application number: 06797475.8
(22) Date of filing: 05.09.2006
(51) Int. Cl.: A61B 90/00, A61B 10/02, A61B 1/00

(54) **TISSUE BIOPSY NEEDLE DEVICE**
NADELVORRICHTUNG FÜR GEWEBEBIOPSIE
DISPOSITIF A AIGUILLE POUR BIOPSIE TISSULAIRE

(30) Priority: 06.09.2005 JP 2005258242
(43) Date of publication of application: 21.05.2008
(73) Proprietor: Olympus Corporation, Tokyo 192-8507 (JP)
(72) Inventor: KODAMA, Hiroshi, Hachioji-shi, Tokyo 192-8507 (JP)
(74) Representative: von Hellfeld, Axel
(86) International application number: PCT/JP2006/317575
(87) International publication number: WO 2007/029713

(56) References cited:
- WO-A2-03/026509
- JP-A- H0 975 351
- JP-A- 06 197 898
- JP-A- 09 075 351
- JP-A- 2001 037 765
- JP-A- 2004 121 852
- US-A- 5 551 442
- US-A1- 2004 068 231

## Description

### Technical Field

The present invention relates to a tissue biopsy needle apparatus, and more particularly, to a tissue biopsy needle apparatus used to puncture a subject site in a body cavity and take a sample of body tissue by being passed into a treatment instrument passage channel of an endoscope inserted into a body cavity.

### Background Art

Recently, a series of medical acts has found a general use which involves acquiring ultrasonic tomographic images of organs in a body cavity using an ultrasonic endoscope inserted in the body cavity, puncturing a body tissue with a puncture needle and taking samples of the body tissue while watching the ultrasonic tomographic images, and conducting a pathologic diagnosis using the tissue samples.

The tissue biopsy needle apparatus configured to take samples of body tissue by watching ultrasonic tomographic images by means of an ultrasonic endoscope is used to take a sample of body tissue by passing a puncture needle into a treatment instrument passage channel provided in an insertion portion of the ultrasonic endoscope inserted in the body cavity and puncturing a target site of an organ in the body cavity with a distal end of the puncture needle which is projected from a distal end of the insertion portion while watching the ultrasonic tomographic images.

With the tissue biopsy needle apparatus, when puncturing a target site of an organ in the body cavity with the distal end of the puncture needle, a surgeon moves the puncture needle back and forth manually. However, attempts to puncture a target site manually with the distal end of the puncture needle may fail if the target site is hard. That is, during an attempt to puncture the hard target site with the puncture needle, the puncture needle pushed in manually can pass by the target site making it impossible as a result to puncture the target site.

Also, it is known that a hard target site which is difficult to puncture with a puncture needle can be punctured more easily if the puncture needle is stuck at high speed. Generally, it is said that the ease with which a target site is punctured increases with speed at which body tissue is punctured with a puncture needle.

Thus, for example, a puncture needle apparatus has been devised which punctures a target site with a puncture needle at high speed using an urging force of a spring member incorporated into an ejection mechanism of the puncture needle.

The puncture needle apparatus configured with an urging spring member to eject the puncture needle at high speed can cause malfunctions due to misoperations such as puncturing body tissue at an unintended site due to an operation mistake, puncturing a treatment instrument passage channel of an ultrasonic endoscope with the puncture needle ejected by an operation mistake during passage of the puncture needle into the treatment instrument passage channel of the ultrasonic endoscope, and ejecting the puncture needle by mistake during cleaning of the puncture needle.

Thus, in relation to conventional puncture needle apparatus equipped with an urging spring member to eject the puncture needle at high speed, a puncture needle operation aids have been proposed in Japanese Patent Laid-Open No. 2001-37765 and the like to prevent the puncture needle ready for ejection from being ejected untimely due to a misoperation.

The puncture needle operation aid disclosed in Japanese Patent Laid-Open No. 2001-37765 comprises a puncture needle which is advanceably/retractably passed into a treatment instrument passage channel of an ultrasonic endoscope and stuck into a subject site in a body cavity; an urging mechanism which ejects the puncture needle by an urging force; a restraining mechanism which temporarily restrains the urging force of the urging mechanism; a switch mechanism which lifts restraint imposed by the restraining mechanism; and a safety device which prevents the switch mechanism from going into action when the restraining mechanism is temporarily exerting restraint.

The puncture needle operation aid can avoid misoperations because the safety device disables the switch mechanism which lifts the restraint temporarily imposed on the urging mechanism by the restraining mechanism.

However, although the puncture needle operation aid disclosed in Japanese Patent Laid-Open No. 2001-37765 includes the switch mechanism which lifts the restraint temporarily imposed by the restraining mechanism on the urging force of the urging mechanism which urges the puncture needle and the safety device which disables the switch mechanism, if a surgeon forgets to disable the switch mechanism by means of the safety device with the urging force of the urging mechanism restrained temporarily by the restraining mechanism and touches or operates the switch mechanism by mistake during insertion of the puncture needle into the insertion portion of the ultrasonic endoscope, during passage of the puncture needle through a non-target site, or during cleaning, the puncture needle can puncture the non-target site or the treatment instrument passage channel of the ultrasonic endoscope or a puncturing operation can occur during cleaning.

JP H09 75351 A describes a needle apparatus wherein an inside needle sliding body is rapidly advanced forward and an inside needle is fired by the effect of a spring means when an inside needle detaining pawl is released. An outside needle detaining pawl is released when the inside needle sliding body comes into contact with a projection of a crank mechanism. The outside needle sliding body is then rapidly advanced forward and an outside needle is fired. A needle tip at a rear end of the inside needle penetrates a cap of a vacuum taking pipe and an opening at a rear end near the needle tip at the rear end is exposed into the vacuum taking pipe and a negative pressure of the vacuum taking pipe is transmitted to an opening at a front end of the inside needle. Tissues sucked from the opening at the front end of the inside needle are excised by a cylindrical needle tip of the outside needle and a tissue piece is taken into the vacuum taking tube via an inside hole of the inside needle.

The present invention has been made in view of the above circumstances and has an object to provide a tissue biopsy needle apparatus which can reliably stop a puncture needle ready to be ejected at high speed with an urging member compressed for an urging force and thereby prevent a surgeon from ejecting the puncture needle at high speed by mistake.

### Disclosure of Invention

### Means for Solving the Problem

This object is addressed by a tissue biopsy needle apparatus according to claim 1.

A tissue biopsy needle apparatus may comprise: ejecting means which, being formed of an elastic member, generates an ejection force to eject a needle tube; drawing means which draws the ejecting means to a compressed position to eject the needle tube; operation means which maintains the ejecting means at a compressed position to which the ejecting means is drawn by the drawing means and releases the ejecting means from the compressed position, thereby ejecting the needle tube; protection means which disables the operation means maintaining the compressed position of the ejecting means drawn by the drawing means; and moving means which moves the protection means from a working position at which the operation means is enabled to a non-working position at which the operation means is disabled in accordance with drawings of the ejecting means to the compressed position by the drawing means.

### Brief Description of the Drawings

Fig. 1 is a top view showing an external configuration of an operation portion of a tissue biopsy needle apparatus according to an embodiment of the present invention;
Fig. 2 is a side view showing the external configuration of the operation portion of the tissue biopsy needle apparatus according to the embodiment of the present invention;
Fig. 3 is a cross-sectional view showing an internal configuration of a handle unit of the tissue biopsy needle apparatus according to the embodiment of the present invention in a preparatory stage for ejection of a needle tube;
Fig. 4 is a cross-sectional view showing an internal configuration of the handle unit of the tissue biopsy needle apparatus according to the embodiment of the present invention before the needle tube is drawn;
Fig. 5 is a plan view showing an inner side of an upper part of a handle body installed in the handle unit of the tissue biopsy needle apparatus according to the embodiment of the present invention;
Fig. 6 is a plan view showing an outer side of the upper part of the handle body installed in the handle unit of the tissue biopsy needle apparatus according to the embodiment of the present invention;
Fig. 7 is a plan view showing an inner side of a lower part of the handle body installed in the handle unit of the tissue biopsy needle apparatus according to the embodiment of the present invention;
Fig. 8 is a plan view showing an outer side of the lower part of the handle body installed in the handle unit of the tissue biopsy needle apparatus according to the embodiment of the present invention;
Fig. 9 is an exploded perspective view showing an internal configuration of the handle unit of the tissue biopsy needle apparatus according to the embodiment of the present invention;
Fig. 10 is an exploded cross-sectional view showing the internal configuration of the handle unit of the tissue biopsy needle apparatus according to the embodiment of the present invention;
Fig. 11 is a plan view showing a configuration of a third tubular body which controls the distal position of the needle tube beyond a sheath by being loosely fitted in the handle unit of the tissue biopsy needle apparatus according to the embodiment of the present invention;
Fig. 12 is a cross-sectional view showing the configuration of the third tubular body which controls the distal position of the needle tube beyond the sheath by being loosely fitted in the handle unit of the tissue biopsy needle apparatus according to the embodiment of the present invention;
Fig. 13 is perspective view showing a configuration of a protective case which protects an operation switch installed in the handle unit of the tissue biopsy needle apparatus according to the embodiment of the present invention;
Fig. 14 is a cross-sectional view showing a relationship between the operation switch and protective case, the operation switch being used to temporarily restrain a drawing tube which compresses an urging member to apply an urging force to the needle tube installed in the handle unit of the tissue biopsy needle apparatus according to the embodiment of the present invention, where the drawing tube is not restrained;
Fig. 15 is a cross-sectional view showing a relationship between the operation switch and protective case, the operation switch being used to temporarily restrain the drawing tube which compresses the urging member to apply an urging force to the needle tube installed in the handle unit of the tissue biopsy needle apparatus according to the embodiment of the present invention, where the drawing tube is restrained;
Fig. 16 is a plan view of a lateral surface of the tissue biopsy needle apparatus according to the embodiment of the present invention, showing an operation of the handle unit in preparation for ejection of the needle tube;
Fig. 17 is a plan view of the lateral surface of the tissue biopsy needle apparatus according to the embodiment of the present invention, showing an operation of inserting the tissue biopsy needle apparatus into an ultrasonic endoscope;
Fig. 18 is a plan view of the lateral surface of the tissue biopsy needle apparatus according to the embodiment of the present invention, showing an operation of the protective cover in the handle unit before ejection of the needle tube;
Fig. 19 is a plan view of the lateral surface of the handle unit in the tissue biopsy needle apparatus according to the embodiment of the present invention, showing an operation of the handle unit after ejection of a puncture needle;
Fig. 20 is a cross-sectional view showing an internal configuration of a needle tube in a handle unit of a tissue biopsy needle apparatus according to a variation of the embodiment of the present invention in a preparatory stage for ejection;
Fig. 21 is a cross-sectional view showing a relationship between an operation switch and protective case, the operation switch being used to temporarily restrain a drawing tube which compresses an urging member to apply an urging force to the needle tube installed in the handle unit of the tissue biopsy needle apparatus according to the variation of the embodiment of the present invention, where the drawing tube is not restrained;
Fig. 22 is a cross-sectional view showing a relationship between the operation switch and protective case, the operation switch being used to temporarily restrain the drawing tube which compresses the urging member to apply an urging force to the needle tube installed in the handle unit of the tissue biopsy needle apparatus according to the variation of the embodiment of the present invention, where the drawing tube is restrained; and
Fig. 23 is a cross-sectional view showing a relationship between the operation switch and protective case, the operation switch being used to temporarily restrain the drawing tube which compresses the urging member to apply an urging force to the needle tube installed in the handle unit of the tissue biopsy needle apparatus according to the variation of the embodiment of the present invention, where a protective cover is pushed out with the drawing tube restrained.

### Best Mode for Carrying Out the Invention

The present invention will be described below with reference to an illustrated embodiment.

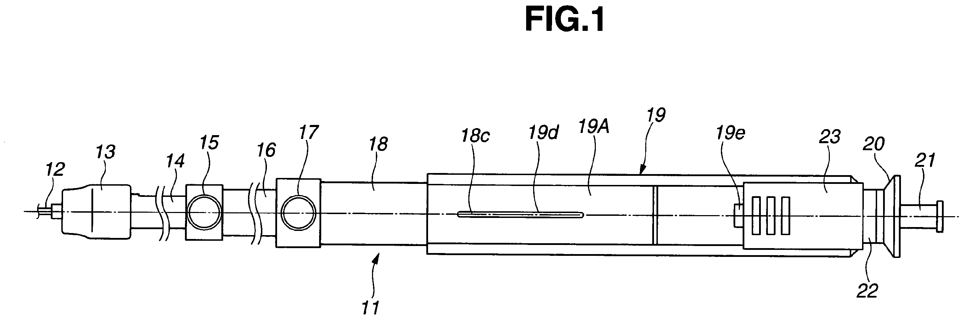

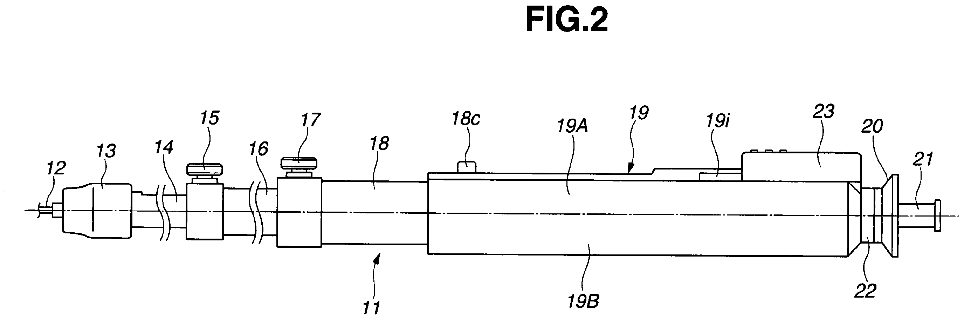

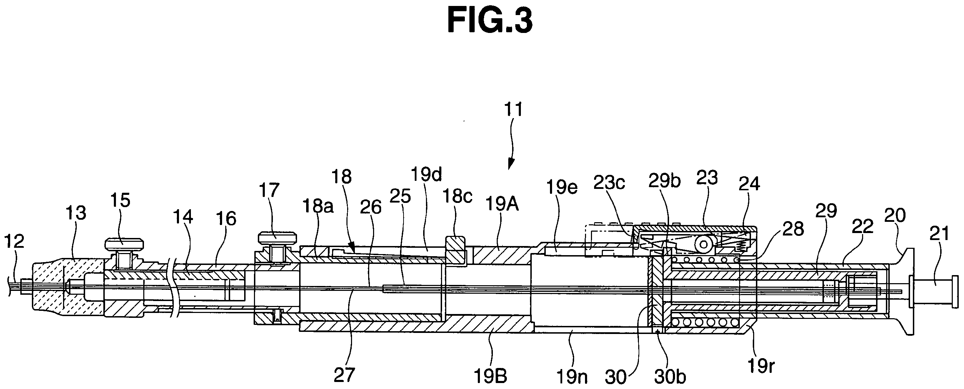

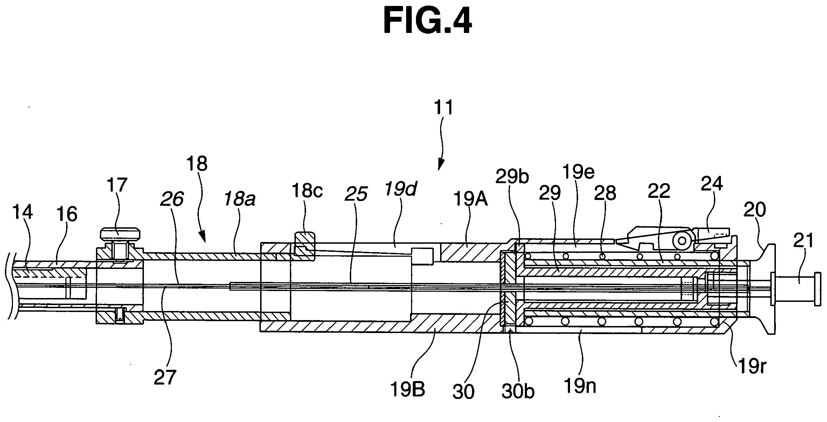

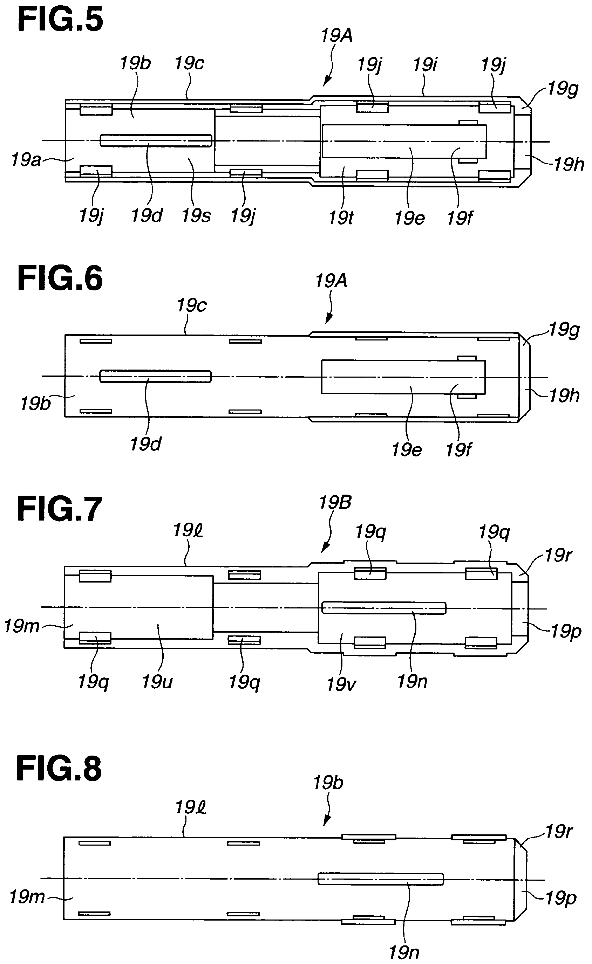

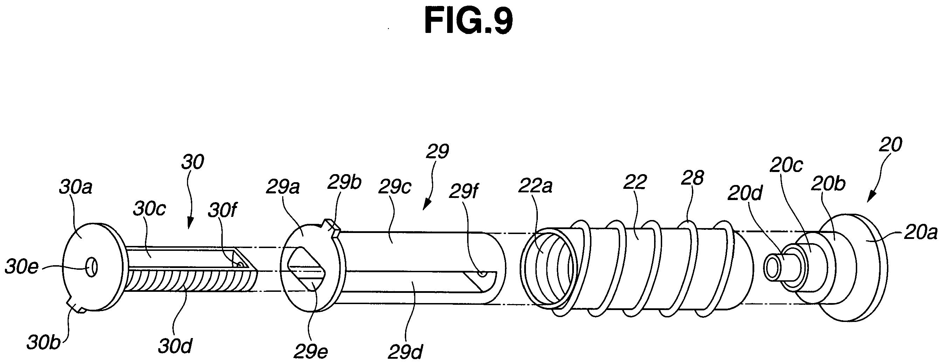

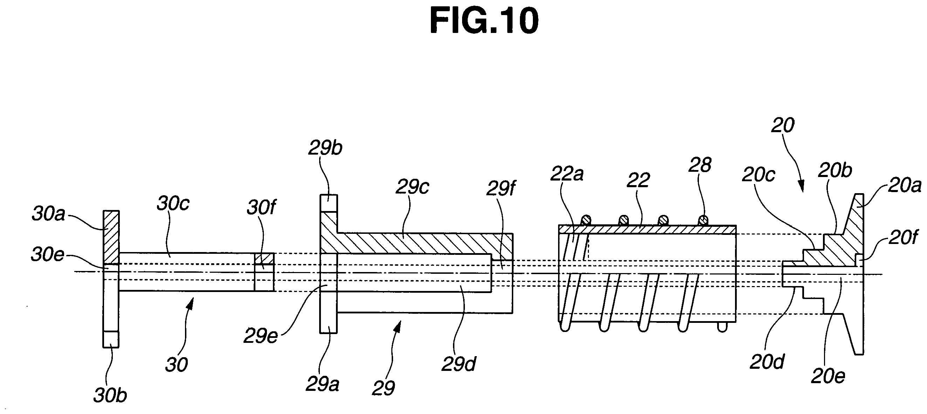

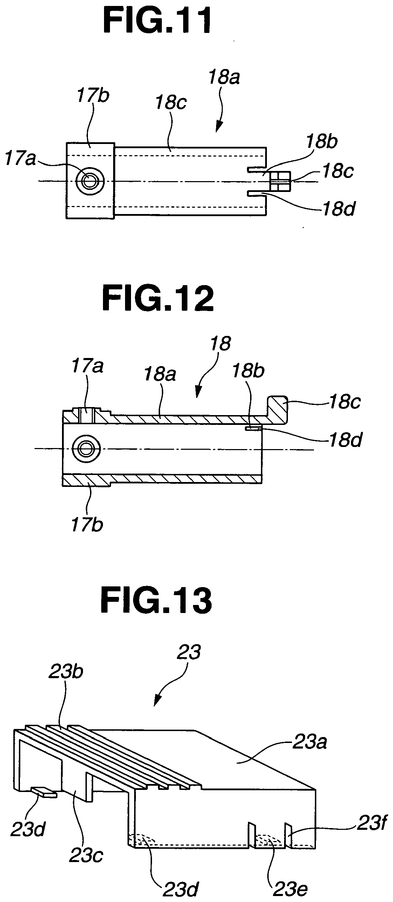

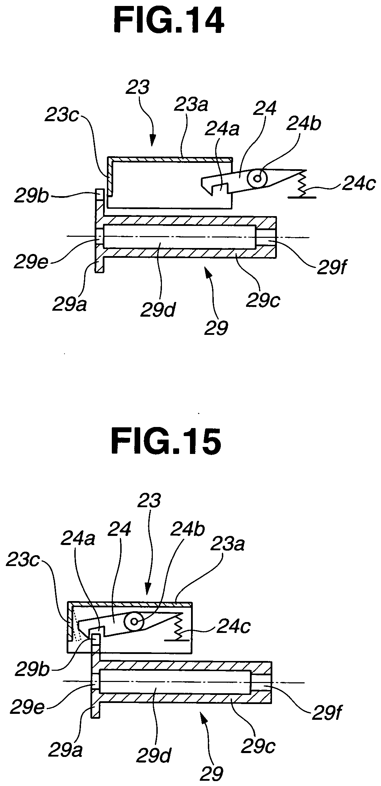

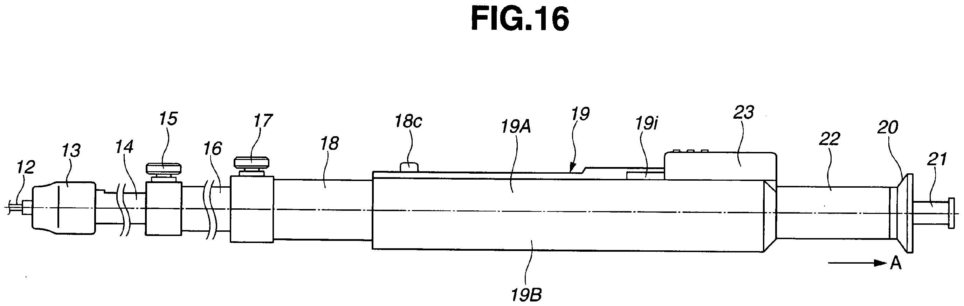

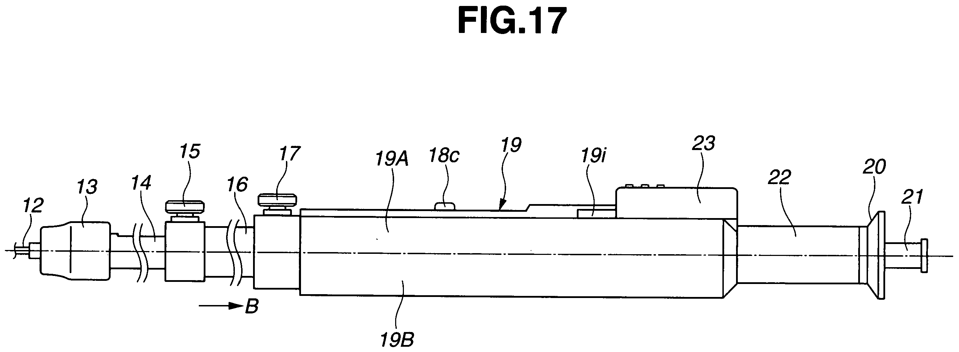

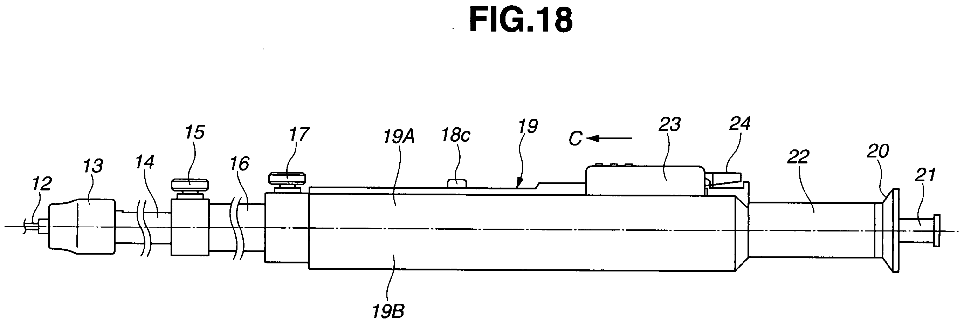

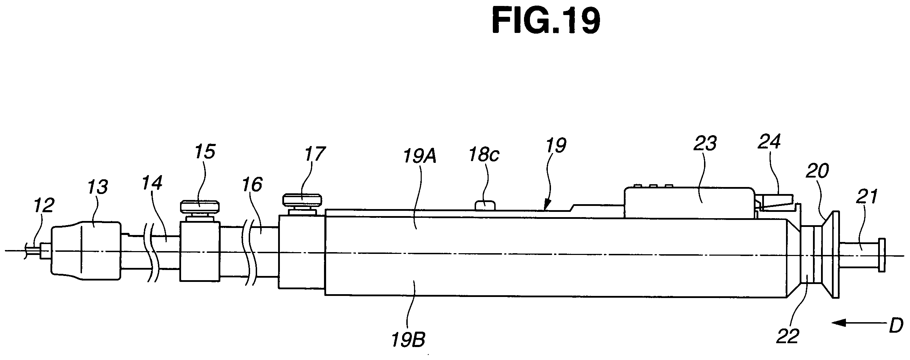

Figs. 1 to 19 show a tissue biopsy needle apparatus according to the embodiment of the present invention. Specifically, Figs. 1 and 2 show an external configuration of an operation portion of the tissue biopsy needle apparatus according to the embodiment of the present invention, where Fig. 1 is a top view and Fig. 2 is a side view. Fig. 3 is a cross-sectional view showing an internal configuration of a handle unit of the tissue biopsy needle apparatus according to the embodiment of the present invention in a preparatory stage for ejection of a puncture needle. Fig. 4 is a cross-sectional view showing an internal configuration of the handle unit of the tissue biopsy needle apparatus according to the embodiment of the present invention before a needle tube is drawn. Figs. 5 and 6 show an upper part of a handle body installed in the handle unit of the tissue biopsy needle apparatus according to the embodiment of the present invention, where Fig. 5 is a plan view showing an inner side and Fig. 6 is a plan view showing an outer side. Figs. 7 and 8 show a lower part of a handle body installed in the handle unit of the tissue biopsy needle apparatus according to the embodiment of the present invention, where Fig. 7 is a plan view showing an inner side and Fig. 8 is a plan view showing an outer side. Figs. 9 and 10 show an internal configuration of the handle unit of the tissue biopsy needle apparatus according to the embodiment of the present invention, where Fig. 9 is an exploded perspective view and Fig. 10 is an exploded cross-sectional view. Figs. 11 and 12 show a configuration of a third tubular body which controls distal position of the needle tube installed in the handle unit of the tissue biopsy needle apparatus according to the embodiment of the present invention, where Fig. 11 is a plan view and Fig. 12 is a cross-sectional view. Fig. 13 is perspective view showing a configuration of a protective case which protects an operation switch installed in the handle unit of the tissue biopsy needle apparatus according to the embodiment of the present invention. Figs. 14 and 15 show a relationship between the operation switch and protective case, the operation switch being used to restrain a drawing tube which compresses an urging member to apply an urging force to the needle tube installed in the handle unit of the tissue biopsy needle apparatus according to the embodiment of the present invention, where Fig. 14 is a cross-sectional view showing a state in which the drawing tube is not restrained and Fig. 15 is a cross-sectional view showing a state in which the drawing tube is restrained. Fig. 16 is a plan view of a lateral surface of the tissue biopsy needle apparatus according to the embodiment of the present invention, showing an operation of the handle unit in preparation for ejection of the needle tube. Fig. 17 is a plan view of the lateral surface of the tissue biopsy needle apparatus according to the embodiment of the present invention, showing an operation of inserting the tissue biopsy needle apparatus into an ultrasonic endoscope. Fig. 18 is a plan view of the lateral surface of the tissue biopsy needle apparatus according to the embodiment of the present invention, showing an operation of the protective cover in the handle unit before ejection of the needle tube. Fig. 19 is a plan view of the lateral surface of the handle unit in the tissue biopsy needle apparatus according to the embodiment of the present invention, showing an operation of the handle unit after ejection of the needle tube.

The external configuration of the tissue biopsy needle apparatus according to the embodiment of the present invention will be described below with reference to Figs. 1 and 2.

The tissue biopsy needle apparatus according to the present embodiment includes a handle unit 11 gripped and operated by a surgeon and a long sheath 12 extended from a distal end of the handle unit 11 and passed into a treatment instrument passage channel in an insertion portion of an ultrasonic endoscope (not shown) through a treatment instrument insertion port in the operation portion of the ultrasonic endoscope, where a needle tube 26 described later is passed into the sheath 12.

The handle unit 11 of the tissue biopsy needle apparatus includes a first tubular body 14 which has a sleeve 13 at a distal end, the sleeve 13 being fitted with a proximal end of the sheath 12 and mounted on the treatment instrument insertion port in the insertion portion of the ultrasonic endoscope; a second tubular body 16 which fits slidably over the first tubular body 14 from a rear end of the first tubular body 14 and has a first fixing screw 15 at a distal end; a third tubular body 18 which fits slidably over the second tubular body 16 from a rear end of the second tubular body 16 and has a second fixing screw 17 at a distal end; a handle body 19 which fits slidably over the third tubular body 18 from a rear end of the third tubular body 18 and incorporates a needle tube ejection mechanism (described later: see a drawing knob 20 and the like in Fig. 1); a drawing knob 20 which is installed at a proximal end of the handle body 19 and used to draw the needle tube ejection mechanism; a needle tube knob 21 inserted into and fitted to the drawing knob 20; and a protective cover 23 which, being installed on a top face of the handle body 19, covers an operation switch 24 (described later: see Figs. 3 and 4).

Incidentally, the drawing knob 20 functions as drawing means used to draw ejecting means (a spring 28 described later) to a compressed position, for example, to eject the needle tube 26.

An internal configuration of the handle unit 11 will be described with reference to Fig. 3.

The first tubular body 14 is fitted in the second tubular body 16 in such a way as to be slidable in a longitudinal direction and sliding position of the first tubular body 14 and second tubular body 16 can be locked by the first fixing screw 15. The sleeve 13 at the distal end of the first tubular body 14 is mounted and fixed on the treatment instrument insertion port installed in the operation portion of the ultrasonic endoscope. The needle tube 26 passed through a hollow portion of the first tubular body 14 passes through the sheath 12 whose proximal end is fixed.

The second tubular body 16 is fitted in the third tubular body 18 in such a way as to be slidable in the longitudinal direction and sliding position of the second tubular body 16 and third tubular body 18 can be locked by the second fixing screw 17. The third tubular body 18 is fitted in the handle body 19 in such a way as to be slidable in the longitudinal direction.

As shown in Figs. 11 and 12, the third tubular body 18 includes a fixing portion 17b in which a female thread portion 17a is formed to mate with the second fixing screw 17, a tubular portion 18a slidably fitted into the handle body 19, an extension strip 18b which extends in the longitudinal direction from part of an outside portion of a proximal end of the tubular portion 18a, a guide strip 18c erected from the extension strip 18b to outside the tubular portion 18a, and a kerf 18d which makes the extension strip 18b elastically swingable in a direction of a center axis of the tubular portion 18a.

As shown in Figs. 1 and 2, the handle body 19 is fitted slidably over the third tubular body 18 and comprises an upper part 19A and lower part 18B which are dividable. The upper part 19A of the handle body 19 has a flat top face. A guide hole 19d in which the guide strip 18c of the third tubular body 18 is inserted to guide sliding is provided at a distal end and the protective cover 23 is placed at a rear end. To install the protective cover 23 slidably, sliding grooves 19i are formed in flanks near the top face of the upper part 19A on which the protective cover 23 is installed.

More specifically, the upper part 19A has a substantially semicircular portion 19a shown in Fig. 5 on an inner side as well as a planar top portion 19b and a flank 19c shown in Fig. 6 on an outer side. The upper part 19A has an approximately concave, long cross section.

A first mounting portion 19s and the first guide hole 19d are formed in the longitudinal direction at the distal end of the upper part 19A, where an outer periphery of the third tubular body 18 is mounted on the first mounting portion 19s and the guide strip 18c (see Figs. 1 and 2) of the third tubular body 18 is inserted into the first guide hole 19d to guide sliding.

The first guide hole 19d is formed in the center of the longitudinal direction between the first mounting portion 19s of the semicircular portion 19a and the top portion 19b.

A second mounting portion 19t, second guide hole 19e, and switch mounting hole 19f are formed in the longitudinal direction at the rear end of the semicircular portion 19a of the upper part 19A, where the needle tube ejection mechanism (described later: the drawing knob 20 and the like) is mounted on the second mounting portion 19t, an elastic tongue 23c (described later) and abutting strip 29b are inserted in the second guide hole 19e to guide sliding, the elastic tongue 23c being an elastic member which serves as an abutting portion extending from the protective cover 23 while the abutting strip 29b combining an engaging insertion portion of a drawing tube 29 (see Fig. 3), and the switch mounting hole 19f in which the operation switch 24 (described later: see Fig. 3) is mounted is communicated with the second guide hole 19e.

The sliding grooves 19i (see Figs. 5 and 2) along which the protective cover 23 slides are formed in the flank 19c of the upper part 19A near where the second guide hole 19e and switch mounting hole 19f are installed.

A shoulder 19g and a tube mounting portion 19h which is semicircular in shape are formed at an extreme rear end of the upper part 19A in the longitudinal direction, where the shoulder 19g restrains a proximal end of a spring 28 which is an elastic member serving as ejecting means and fitted loosely over a spring tube 22 (see Fig. 3) of the needle tube ejection mechanism (described later) and an outer periphery of the spring tube 22 is slidably mounted on the tube mounting portion 19h.

A plurality of mating strips 19j are formed at equal intervals on the flank 19c of the upper part 19A on a distal side to mate the upper part 19A with the lower part 19B.

The operation switch 24 is mounted in the switch mounting hole 19f of the upper part 19A as shown in Figs. 3, 4, 14, and 15.

As shown in Figs. 14 and 15, the operation switch 24 has a hook-shaped restraining portion 24a and a spring member 24c at opposite ends across a rotational axis 24b, the restraining portion 24a restraining the abutting strip 29b (see Fig. 3) of the drawing tube 29 (described later) and the spring member 24c constantly urging the restraining portion 24a counterclockwise around the rotational axis 24b in the figure.

The operation switch 24 is designed such that the restraining portion 24a urged by the spring member 24c will restrain the abutting strip 29b of the drawing tube 29 and that when the other end of the operation switch 24 is pushed down against the urging of the spring member 24c, the restraining portion 24a rotates clockwise around the rotational axis 24b in the figure, lifting the restraint on the abutting strip 29b of the drawing tube 29.

The lower part 19B of the handle body 19 is a long, semicircular body 191 which has a semicircular cross section as shown in Figs. 7 and 8. A semicircular portion 19m on an inner peripheral side of the cylindrical body 191 of the lower part 19B has a third mounting portion 19u formed at the distal end in the longitudinal direction, and a fourth mounting portion 19v formed at the rear end, where the outer periphery of the third tubular body 18 is mounted on the third mounting portion 19u and the needle tube ejection mechanism (described later) is mounted on the fourth mounting portion 19v. A third guide hole 19n in which a guide strip 30b (see Fig. 3) of an ejection adjustment tube 30 of the needle tube ejection mechanism is mounted to guide sliding is formed in the fourth mounting portion 19v. A shoulder 19r and a tube mounting portion 19p which is semicircular in shape are formed at an extreme rear end of the semicircular body 191 of the lower part 19B in the longitudinal direction, where the shoulder 19r restrains the proximal end of a spring 28 which is an elastic member serving as ejecting means and fitted loosely over the spring tube 22 of the needle tube ejection mechanism (described later) and the outer periphery of the spring tube 22 is slidably mounted on the tube mounting portion 19p. A plurality of mating holes 19q are formed at equal intervals at the distal end of the semicircular body 191 of the lower part 19B to mate with the mating strips 19j of the upper part 19A.

That is, being mounted on the outer periphery of the third tubular body 18 as shown in Figs. 3 and 4, the first mounting portion 19s and third mounting portion 19u at the distal end of the upper part 19A and lower part 19B of the handle body 19 mate the mating strips 19j of the upper part 19A with the mating holes 19q in the lower part 19B. At this time, as the guide strip 18c of the third tubular body 18 is fitted in the first guide hole 19d in the upper part 19A, the third tubular body 18 is mounted on the handle body 19 in such a way as to be slidable in the longitudinal direction.

The second mounting portion 19t and fourth mounting portion 19v at the rear end of the upper part 19A and lower part 19B of the handle body 19 incorporate the needle tube ejection mechanism which ejects the needle tube 26 as shown in Figs. 3 to 6.

As shown in Figs. 3 and 4, the needle tube ejection mechanism includes the needle tube knob 21, drawing knob 20, spring tube 22, drawing tube 29, ejection adjustment tube 30, operation switch 24, and protective cover 23, where the needle tube knob 21 is installed at the proximal end of the needle tube 26 and a stylet 27 which is passed into the needle tube 26, the drawing knob 20 serving as part of drawing means has a passage hole in a center into which the needle tube 26 with the stylet 27 passed is passed and in which the needle tube knob 21 is fitted and fixed, the spring tube 22 is an elastic-member tube over which the coiled spring 28 is fitted, the drawing tube 29 is loosely and slidably fitted in the spring tube 22, a proximal end of the drawing tube 29 is screwed into the drawing knob 20 while a distal end of the drawing tube 29 abuts a distal end of the spring 28, the ejection adjustment tube 30 adjusts sliding distance of the drawing tube 29 due to the spring 28, i.e., ejection distance of the needle tube 26, by being loosely fitted in the drawing tube 29, the operation switch 24 serving as operation means is used to maintain compressed position when the spring 28 is compressed for ejection of the needle tube 26 at high speed by operating the drawing knob 20 and eject the needle tube 26 by releasing the spring 28 from the compressed position, and the protective cover 23 serving as protection means covers and thereby disables the operation switch 24 to prevent the operation switch 24 from being operated unnecessarily at the compressed position of the spring 28.

Incidentally, the drawing means includes the drawing knob 20, the spring tube 22 which is loosely fitted with the spring 28, and the drawing tube 29 which is fitted in the spring tube 22, restraining one end of the spring 28, and is drawn against elastic force of the spring 28 by means of the drawing knob 20.

Now, configurations of the drawing knob 20, spring tube 22, drawing tube 29, and ejection adjustment tube 30 will be described in detail with reference to Figs. 9 and 10.

The drawing knob 20 includes a knob 20a which is approximately disk-shaped, a first shoulder 20b which is smaller in outside diameter than the knob 20a and is inserted in the spring tube 22, a second shoulder 20c which is smaller in outside diameter than the first shoulder 20b, and a male thread portion 20d which is smaller in outside diameter than the second shoulder 20c and on which male threads to be screwed into the drawing tube 29 are formed. A conduit 20e for passage of the needle tube 26 is formed along a center axis of the knob 20a, first shoulder 20b, second shoulder 20c, and male thread portion 20d. A female thread portion 20f into which the needle tube knob 21 is screwed is formed on a proximal end face of the knob 20a.

The spring tube 22 is a cylindrical body over which the coiled spring 28 is fitted. A female thread portion 22a into which a male thread portion 30d of the ejection adjustment tube 30 is screwed is formed on an inner periphery of the spring tube 22 at the distal end. The first shoulder 20b of the drawing knob 20 is inserted and fitted in the spring tube 22 at the proximal end.

The drawing tube 29 includes a closed-end cylinder 29c loosely fitted in the spring tube 22, a collar 29a circular in shape and installed at a distal end of the closed-end cylinder 29c; a pair of rectangular windows 29d formed by cutting away rectangular areas from opposing arched side walls of the closed-end cylinder 29c; a female thread portion 29f into which the male thread portion 20d of the drawing knob 20 is screwed, the drawing knob 20 being formed in an axial center of a distal-side bottom face of the closed-end cylinder 29c; an insertion hole 29e formed in a center of the collar 29a in such a form as to allow passage of the ejection adjustment tube 30; the abutting strip 29b which abuts the elastic tongue 23c of the protective cover 23 (described later) extending from part of an outer periphery of the collar 29a.

The ejection adjustment tube 30 includes a barrel-shaped tube 30c which has a barrel-shaped cross section, being formed by removing opposing arched side walls of a tubular body equal in outside diameter to the closed-end cylinder 29c of the drawing tube 29 in the longitudinal direction; a collar 30a circular in shape and installed at a distal end of the barrel-shaped tube 30c; a male thread portion 30d formed on an outer periphery of the barrel-shaped tube 30c; a needle tube passage hole 30e formed in a center of the collar 30a of the ejection adjustment tube 30; a needle tube passage hole 30f formed in a center of a proximal-side bottom face; a guide strip 30b extending from part of an outer periphery of the collar 30a and slidably fitted in the third guide hole 19n in the lower part 19B of the handle body 19.

The rectangular windows 29d of the drawing tube 29 and the male thread portion 30d of the ejection adjustment tube 30 are designed to be nearly identical in shape and the closed-end cylinder 29c of the drawing tube 29 and the male thread portion 30d of the ejection adjustment tube 30 are designed to be nearly equal in diameter so that the male thread portion 30d will fit in the rectangular windows 29d.

The needle tube ejection mechanism which includes the drawing knob 20, spring tube 22, drawing tube 29, and ejection adjustment tube 30 described above is assembled as follows.

Specifically, a proximal end of the barrel-shaped tube 30c of the ejection adjustment tube 30 is inserted into the closed-end cylinder 29c through the insertion hole 29e formed in the collar 29a of the drawing tube 29. Consequently, the male thread portion 30d of the ejection adjustment tube 30 inserted into the drawing tube 29 is located on a same outside diameter as the closed-end cylinder 29c, being exposed from the rectangular windows 29d of the drawing tube 29. Incidentally upon inserting the ejection adjustment tube 30 into the drawing tube 29, the guide strip 30b of the collar 30a of the ejection adjustment tube 30 is placed in opposing relation to the abutting strip 29b of the collar 29a of the drawing tube 29.

Next, the drawing tube 29 with the ejection adjustment tube 30 inserted is fitted in the spring tube 22, screwing the male thread portion 30d of the ejection adjustment tube 30 into the female thread portion 22a on the inner periphery of the spring tube 22. That is, the male thread portion 30d formed outside the barrel-shaped tube 30c of the ejection adjustment tube 30 loosely fitted in the drawing tube 29 is located in the rectangular windows 29d of the drawing tube 29 and is thus screwed into the female thread portion 22a of the spring tube 22.

When the drawing tube 29 with the ejection adjustment tube 30 loosely fitted is fitted in the spring tube 22 and the male thread portion 30d of the ejection adjustment tube 30 is screwed into the female thread portion 22a, the first shoulder 20b of the drawing knob 20 is inserted through a proximal end the spring tube 22 and consequently the male thread portion 20d at a distal end of the drawing knob 20 is screwed into the female thread portion 29f at a proximal end the drawing tube 29.

The needle tube ejection mechanism thus assembled is incorporated into the handle body 19 described with reference to Figs. 5 to 8. To incorporate the needle tube ejection mechanism into the handle body 19 (see Figs. 3 to 8), the third tubular body 18 is mounted in the third mounting portion 19u at a distal end of the semicircular portion 19m inside the lower part 19B of the handle body 19, the collar 30a of the ejection adjustment tube 30 is mounted on the fourth mounting portion 19v at a rear end, and the guide strip 30b installed on the collar 30a is fitted in the third guide hole 19n in the lower part 19B.

Furthermore, a proximal end of the spring 28 of the spring tube 22 is placed inside the shoulder 19r at the proximal end of the lower part 19B while an outer periphery of a proximal end of the spring tube 22 is placed on the tube mounting portion 19p of the lower part 19B.

Next, the upper part 19A equipped with the operation switch 24 of the handle body 19 is mounted on the lower part 19B. Assembly of the upper part 19A involves mounting the third tubular body 18 on the first mounting portion 19s at the distal end of the upper part 19A, fitting the guide strip 18c of the third tubular body 18 in the first guide hole 19d, on the rear end side mounting the collar 29a of the drawing tube 29 on the second mounting portion 19t, and mounting the abutting strip 29b of the collar 29a in the second guide hole 19e. Furthermore, the proximal end of the spring 28 of the spring tube 22 is placed inside the shoulder 19g at the proximal end of the upper part 19A while the outer periphery of the proximal end of the spring tube 22 is mounted on the tube mounting portion 19h of the upper part 19A.

That is, the third tubular body 18 as well as the needle tube ejection mechanism which includes the drawing knob 20, spring tube 22, drawing tube 29, and ejection adjustment tube 30 are incorporated into the upper part 19A and lower part 19B, the mating strips 19j of the upper part 19A are mated with the mating holes 19q in the lower part 19B, and the needle tube 26 is inserted through the conduit 20e of the drawing knob 20. Consequently, the needle tube 26 is passed through the conduit 20e of the drawing knob 20; a hollow portion of the spring tube 22; the female thread portion 29f of the drawing tube 29; the needle tube passage holes 30f and 30e of the ejection adjustment tube 30; and hollow portions of the third tubular body 18, the second tubular body 16, and the first tubular body 14; and into the sheath 12 whose proximal end is connected with the sleeve 13.

As shown in Fig. 4, with the needle tube ejection mechanism incorporated into the handle body 19, when the drawing knob 20 is drawn, the drawing tube 29 screwed onto the male thread portion 20d of the drawing knob 20 is drawn as well. When the drawing tube 29 is drawn, the ejection adjustment tube 30 screwed in the spring tube 22 loosely fitted over the drawing tube 29 is drawn together with the spring tube 22. That is, when the drawing knob 20 is drawn, the spring 22 is compressed against urging of the spring 22, drawing the spring tube 22, drawing tube 29, and ejection adjustment tube 30 all together.

During the drawing, the ejection adjustment tube 30 is drawn as the guide strip 30b of the collar 30a is guided by the third guide hole 19n in the lower part 19B while the drawing tube 29 is drawn as the abutting strip 29b of the collar 29a is guided by the second guide hole 19e in the upper part 19A. When the collar 29a which is an abutting portion of the drawing tube 29 is drawn to a position where it is engaged with the restraining portion 24a of the operation switch 24, the restraining portion 24a of the operation switch 24 is restrained to the collar 29a by the spring member 24c and thereby enters a state shown in Figs. 3 and 15, making the needle tube 26 ready for ejection. When the other end of the operation switch 24 is pushed down against the spring member 24c, the abutting strip 29b of the drawing tube 29 is released from the restraining portion 24a. Consequently, the spring 28 decompresses, causing the spring tube 22, ejection adjustment tube 30, drawing knob 20, and needle tube knob 21 attached to the drawing knob 20 to move at high speed, and consequently a needle point of the needle tube 26 whose proximal end is attached to the needle tube knob 21 is ejected through the distal end of the sheath 12 at high speed.

Next, the protective cover 23 which prevents the operation switch 24 from being operated inadvertently will be described with reference also to Fig. 13.

The protective cover 23 which prevents the operation switch 24 installed on the upper part 19A of the handle body 19 from being operated inadvertently includes at least a rectangular member 23a which is U-shaped in cross section to cover the operation switch 24, a concavo-convex portion 23b intended to prevent skidding for the surgeon who touches a top face of the rectangular member 23a, the elastic tongue 23c extending vertically from a center of one opening of the rectangular member, a plurality of sliding strips 23d and 23e fitted in the sliding grooves 19i of the upper part 19A from inside both flanks of the rectangular member 23a, and notches 23f formed on both sides of the sliding strips 23e.

The protective cover 23 shaped as described above has the sliding strips 23d mounted in the second guide hole 19e in the upper part 19A of the handle body 19 and the sliding strips 23d and 23e fitted in the sliding grooves 19i which are formed in the flanks of the upper part 19A. That is, the protective cover 23 is supported from both sides by means of the sliding strips 23d and 23e in the sliding grooves 19i provided in the flanks of the upper part 19A so that it will not be detached easily from the upper part 17a. Furthermore, although not illustrated, a protrusion is provided at an end point of sliding of the protective cover 23 in the sliding grooves 19i of the upper part 19A so that the sliding strips 23d and 23e can fit and remain stopped and that a click can be felt when the protective cover 23 is manipulated.

In the protective cover 23 thus installed in the upper part 19A, the drawing tube 29 is pushed out toward the distal end under an urging force of the spring 28 as shown in Fig. 14, but when the drawing tube 29 is drawn against the urging of the spring 28 drawn by the drawing knob 20 as described above, the abutting strip 29b of the collar 29a of the drawing tube 29 abuts the elastic tongue 23c of the protective cover 23, thereby drawing the protective cover 23.

That is, the elastic tongue 23c serves as moving means which moves the protective cover 23 from a working position where the operation switch 24 is enabled to a non-working position where the operation switch 24 is disabled, as the spring 28 is drawn by the drawing knob 20 to the compressed position.

The working position where the operation switch 24 is enabled by the protective cover 23 is a position where operation of the operation switch 24 is unprevented by the protective cover 23.

The non-working position where the operation switch 24 is disabled by the protective cover 23 is a position where the operation of the operation switch 24 is prevented by the protective cover 23.

When the protective cover 23 is drawn to a position where it covers the operation switch 24 at an end point of the sliding grooves 19i in the upper part 19A, the elastic tongue 23c of the protective cover 23 is deformed as indicated by a dotted line in Fig. 15, separating from the abutting strip 29b of the drawing tube 29 and leaving the protective cover 23 in place. After separating from the elastic tongue 23c of the protective cover 23, the drawing tube 29 continues drawing and is inserted and engaged with the restraining portion 24a of the operation switch 24.

In the configuration in which the collar 29a is an elastic member, after the protective cover 23 is moved to the non-working position of the operation switch 24 with the abutting strip 29b abutted against the collar 29 by means of the drawing knob 20, further drawing by the drawing knob 20 causes the collar 29a to be elastically deformed, separating the drawing knob 20 from the protective cover 23 and leaving the protective cover 23 at the non-working position of the operation switch 24.

Consequently, when the drawing tube 29 is drawn together with the needle tube 26 against the urging of the spring 28 by means of the drawing knob 20 in preparation for ejection of the needle tube 26, the protective cover 23 is drawn as well by the drawing tube 29, covering the operation switch 24 and causing the operation switch 24 to restrain the drawing tube 29. To eject the needle tube 26, the surgeon pushes out the protective cover 23 which covers the operation switch 24 toward the distal end of the upper part 19A thereby exposing the operation switch 24 and pushes down the operation switch 24 against the spring member 24c. Consequently, the restraining portion 24a of the operation switch 24 is disengaged from the abutting strip 29b, causing the drawing tube 29 to slide at high speed under the urging force of the spring 28 toward the distal end, i.e., in such a direction as to eject the needle tube 26.

A sliding stroke of the drawing tube 29, i.e., an ejection stroke from the distal end of the sheath 12 of the needle tube 26 can be adjusted using mating position between the male thread portion 30d of the ejection adjustment tube 30 and female thread portion 22a of the spring tube 22. That is, since a sliding stroke of the ejection adjustment tube 30 is limited by the third guide hole 19n in the lower part 19B, the sliding stroke becomes shorter when the ejection adjustment tube 30 is inserted deep into the spring tube 22 and the sliding stroke becomes longer when the ejection adjustment tube 30 is inserted shallowly into the spring tube 22. This makes it possible to adjust the ejection stroke from the distal end of the sheath 12 of the needle tube 26.

Incidentally, the pair of opposing rectangular windows 29d are formed in the flanks of the drawing tube 29, extending in the longitudinal direction. The ejection adjustment tube 30 is installed inside the drawing tube 29, being equipped with the male thread portion 20d mounted advanceably/retractably and exposed through the rectangular windows 29d and thereby allowing the ejection distance of the needle tube 26 to be adjusted through adjustment of the mating position between the male thread portion 20d on the ejection adjustment tube 30 and the female thread portion 29f on the inner periphery of the ejection adjustment tube 30.

Also, since the male thread portion 30d of the ejection adjustment tube 30 is passed into the closed-end cylinder 29c of the drawing tube 29, resulting in a single outside diameter, it is possible to reduce combined length of the drawing tube 29 and ejection adjustment tube 30, thereby downsizing the handle unit 11.

As described above, the tissue biopsy needle apparatus according to the present invention comprises: ejecting means including the spring 28 which, being an elastic member, generates an ejection force to eject the needle tube 26; drawing means including the drawing knob 20, spring tube 22, drawing tube 29, and ejection adjustment tube 30 which draw the ejecting means to a compressed position; operation means including the operation switch 24 which maintains the ejecting means at a compressed position to which the ejecting means is drawn by the drawing means and releases the ejecting means from the compressed position, thereby ejecting the needle tube 26; protection means including the protective cover 23 which disables the operation means maintaining the compressed position of the ejecting means drawn by the drawing means; and moving means including the abutting strip 29b of the drawing tube 29 and the elastic tongue 23c on the protective cover 23 which move the protection means from a working position at which the operation means is enabled to a non-working position at which the operation means is disabled as the ejecting means is drawn to the compressed position by the drawing means.

Next a method for operating the tissue biopsy needle apparatus according to the present invention will be described in detail with reference to Figs. 16 to 19.

Before shipment of the tissue biopsy needle apparatus as a product or before sampling of body tissue, at least the distal end of the needle tube 26 must not stick out from the distal end of the sheath 12 and the spring 28 must be in an initial state. Thus, as shown in Figs. 1 and 2, to put the spring 28 in the initial state in which the spring 28 is neither compressed nor stretched, the distal end of the drawing knob 20 on which the needle tube knob 21 is mounted is kept abutted against the proximal end of the handle body 19 and the protective cover 23 is intentionally moved so as to cover the operation switch 24. Furthermore, at least the third tubular body 18 is fully pulled out of the handle body 19 so that the needle tube 26 will not stick out from the distal end of the sheath 12.

Next, when inserting the tissue biopsy needle apparatus into a treatment instrument passage channel to take a sample of body tissue by watching ultrasonic tomographic images using the ultrasonic endoscope, the surgeon draws the drawing knob 20 in a direction of arrow A in Fig. 16. As the surgeon draws the drawing knob 20, the spring tube 22, drawing tube 29, and ejection adjustment tube 30 are drawn against the urging of the spring 28, compressing the spring 28 as described above. The drawing of the drawing knob 20 causes the abutting strip 29b of the drawing tube 29 to abut the elastic tongue 23c of the protective cover 23, causing the protective cover 23 to move and cover the operation switch 24. Also, the restraining portion 24a of the operation switch 24 is engaged with the abutting strip 29b of the drawing tube 29, maintaining the compression of the spring 28. That is, the tissue biopsy needle apparatus is loaded with an ejection force to eject the needle tube 26.

Once the tissue biopsy needle apparatus has been loaded with the ejection force, to take samples of body tissue, the surgeon inserts the tissue biopsy needle apparatus into the treatment instrument passage channel of the ultrasonic endoscope and moves the third tubular body 18 in a direction of arrow B in Fig. 17, i.e., in a direction of insertion into the handle body 19 so that the needle point of the needle tube 26 will stick out from the distal end of the sheath 12. That is, when the tissue biopsy needle apparatus loaded with the ejection force is inserted into the treatment instrument passage channel of the ultrasonic endoscope, since the operation switch 24 is covered with the protective cover 23, there is no possibility that the operation switch 24 will be operated by mistake.

To eject the needle point of the needle tube 26 sticking out from the distal end of the sheath 12 to a target site under observation through the ultrasonic endoscope, the surgeon makes the needle tube 26 stick out from the distal end of the sheath 12 by sliding the first tubular body 14, second tubular body 16, and third tubular body 18 and exposes the operation switch 24 by moving the protective cover 23 in a direction of arrow C as shown in Fig. 18.

When the surgeon presses the exposed operation switch 24, the restraining portion 24a of the operation switch 24 is disengaged from the abutting strip 29b of the drawing tube 29, causing the drawing knob 20 and needle tube knob 21 linked to the drawing tube 29 to move in a direction of arrow D shown in Fig. 19 at high speed under the urging force of the compressed spring 28. Consequently, the needle tube 26 fixed to the needle tube knob 21 at the proximal end is ejected from the distal end of the sheath 12, puncturing the target site.

After the needle point of the needle tube 26 punctures the target site, the tissue biopsy needle apparatus is returned to the same state as before the shipment of the product or before the sampling of body tissue described with reference to Figs. 1 and 2 and withdrawn from the treatment instrument passage channel of the ultrasonic endoscope.

As described above, when the tissue biopsy needle apparatus according to the present invention is loaded with an ejection force to eject the needle tube 26, the surgeon securely covers the operation switch 24 which maintains compressed state of the spring 28 with the protective cover 23 by drawing the drawing knob 20. Thus, when the tissue biopsy needle apparatus according to the present invention is loaded with an ejection force, the switch cannot be operated unless the protective cover 23 covering the operation switch 24 is removed. This prevents the needle tube 26 from being ejected inadvertently.

Incidentally, although according to the embodiment described above, the drawing means is held at the compressed position after the protective cover is removed, the drawing means can be designed to be held with the protective cover attached if the position of the abutting strip is established appropriately. In that case, the protective cover is separated by deforming an elastic member when the protective cover is moved from the non-working position to the working position.

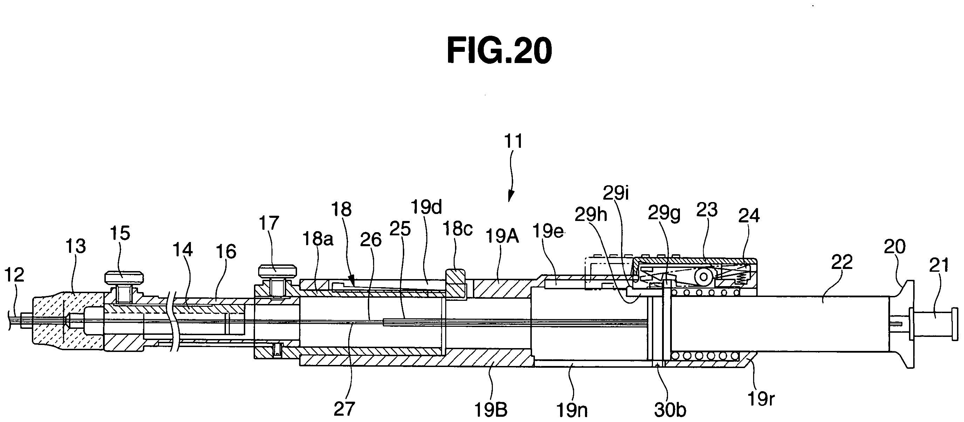

Also, although according to the present embodiment, the elastic tongue 23c of the protective cover 23 is an elastic member, an abutting strip 29h of an elastic member may be installed in an engaging insert 29g inserted and engaged with the restraining portion 24a of the operation switch 24, as in the case of a variation shown in Fig. 20.

Incidentally, an abutting protrusion 29i which abuts laterally against a distal end portion of the protective cover 23 is installed, for example, in a distal end portion of the abutting strip 29h. Also, in Fig. 20, the elastic tongue 23c is removed from the protective cover 23.

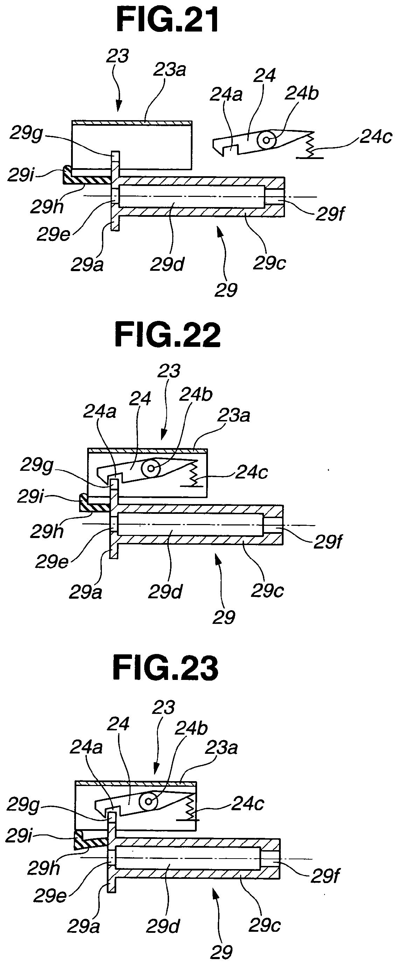

Fig. 20, which shows the variation of the embodiment described above, is a cross-sectional view showing an internal configuration of a needle tube in a handle unit of the tissue biopsy needle apparatus in a preparatory stage for ejection. Figs. 21 to 23 show a relationship between an operation switch and protective case, the operation switch being used to temporarily restrain a drawing tube which compresses an urging member to apply an urging force to a needle tube installed in a handle unit of the tissue biopsy needle apparatus according to the variation of the embodiment, where Fig. 21 is a cross-sectional view showing a state in which the drawing tube is not restrained, Fig. 22 is a cross-sectional view showing a state in which the drawing tube is restrained, and Fig. 23 is a cross-sectional view showing a state in which a protective cover is pushed out with the drawing tube restrained.

With the above-described configuration, when the drawing tube 29 has been pushed out toward the distal end under the urging force of the spring 28, if the drawing tube 29 is drawn by the drawing knob 20 against the urging force of the spring 28, the abutting protrusion 29i of the abutting strip 29h protruding from the collar 29a of the drawing tube 29 abuts laterally against the distal end portion of the protective cover 23 as shown in Fig. 21, drawing the protective cover 23.

When the protective cover 23 is drawn to a position where it covers the operation switch 24 at the end point of the sliding grooves 19i in the upper part 19A, the protective cover 23 is positioned with the abutting protrusion 29i abutted laterally against the distal end portion of the protective cover 23 as shown in Fig. 22. In this state, the engaging insert 29g is inserted and engaged with the restraining portion 24a of the operation switch 24. Thus, when the surgeon draws the drawing knob 20, drawing the drawing tube 29 together with the needle tube 26 against the urging force of the spring 28 to eject the needle tube 26, the protective cover 23 is drawn as well by the drawing tube 29, covering the operation switch 24 and causing the operation switch 24 to restrain the drawing tube 29.

To eject the needle tube 26, the surgeon pushes out the protective cover 23 covering the operation switch 24 toward the distal end of the upper part 19A. Consequently, as shown in Fig. 23, the abutting strip 29h is deformed elastically, separating the protective cover 23 from the drawing tube 29 with a click, and subsequently the protective cover 23 is moved toward the working position. Then, with the operation switch 24 exposed, when an end of the operation switch 24 is pushed down against the spring member 24c, the restraining portion 24a of the operation switch 24 is disengaged from the engaging insert 29g of the drawing tube 29, causing the drawing tube 29 to slide at high speed under the urging force of the spring 28 toward the distal end, i.e., in such a direction as to eject the needle tube 26.

That is, with a configuration in which the abutting strip 29h is an elastic member, when the protective cover 23 is moved to the non-working position of the operation switch 24 with the abutting strip 29h abutted against the abutting protrusion 29i by drawing the drawing knob 20, the protective cover 23 is positioned at the non-working position of the operation switch 24 with the abutting strip 29h and the abutting protrusion 29i abutted against each other. Subsequently, when the protective cover 23 is moved to the working position, the abutting strip 29h is deformed elastically, separating the drawing knob 20 from the protective cover 23.

Thus, the variation shown in Fig. 20 offers the same advantage as that of the embodiment described above.

It should be noted that the present invention is not limited to the embodiment described above, and that various modifications can be made without departing from the scope of the prèsent invention.

This application is based upon and claims the benefit of priority from the prior Japanese Patent Application No. 2005-258242 filed on September 6, 2005.

## Claims

1. A tissue biopsy needle apparatus comprising:
ejecting means which, being formed of an elastic member (28), generates an ejection force to eject a needle tube (26);
drawing means which draws the ejecting means to a compressed position to eject the needle tube (26);
operation means which maintains the ejecting means at a compressed position to which the ejecting means is drawn by the drawing means and releases the ejecting means from the compressed position, thereby ejecting the needle tube (26);
protection means which disables the operation means maintaining the compressed position of the ejecting means drawn by the drawing means; and
moving means which moves the protection means from a working position at which the operation means is enabled to a non-working position at which the operation means is disabled in accordance with drawing of the ejecting means to the compressed position by the drawing means,
**characterized in that**
the moving means includes an abutting portion (23c) extending from a protective cover (23) which is the protective means abutted directly, and an abutting strip (29b) installed on the drawing means which abuts the abutting portion; and
one of the abutting portion (23c) and the abutting strip (29b) is an elastic member.

2. The tissue biopsy needle apparatus according to claim 1, wherein the protective cover (23) which is the protection means has a function to prevent misoperations of the operation means, being placed slidably in relation to the operation means.

3. The tissue biopsy needle apparatus according to claim 1, wherein:
the abutting portion (23c) is an elastic member; and
after the drawing means is drawn, abutting the abutting strip (29b) against the abutting portion (23c) and moving the protective cover (23) to the non-working position of the operation means, when the drawing means is drawn further, the abutting portion (23c) is deformed elastically, separating the drawing means from the protective cover (23) and leaving the protective cover (23) at the non-working position of the operation means.

4. The tissue biopsy needle apparatus according to claim 1, wherein:
the abutting strip (29b) is an elastic member; and
when the drawing means is drawn, abutting the abutting strip (29b) against the abutting portion (23c) and moving the protective cover (23) to the non-working position of the operation means, the protective cover (23) is placed at the non-working position of the operation means with the abutting portion (23c) and the abutting strip (29b) abutted against each other, and when the protective cover (23) is moved to the working position subsequently, the abutting strip (29b) is deformed elastically, separating the drawing means from the protective cover (23).

5. The tissue biopsy needle apparatus according to any one of claims 1 to 4, wherein the working position at which the operation means is enabled by the protection means is a position at which the protection means allows operation of the operation means and the non-working position at which the operation means is disabled by the protection means is a position at which the protection means prevents the operation of the operation means.

6. The tissue biopsy needle apparatus according to any one of claims 1 to 5, wherein the drawing means includes a drawing knob (20), an elastic-member tube (22) loosely fitted with the elastic member (28) which is the ejecting means, and a drawing tube (29) which is fitted in the elastic-member tube (22), restraining one end of the elastic member (28), and is drawn by the drawing knob (20) against an elastic force of the elastic member (28).

7. The tissue biopsy needle apparatus according to claim 6, wherein a pair of opposing rectangular windows (29d) are formed in the flanks of the drawing tube (29) of the drawing means, extending in a longitudinal direction, and an ejection adjustment tube (30) is installed advanceably/retractably in the inner periphery of the drawing tube (29), being equipped with a male thread portion (30d) exposed through the rectangular windows (29d) and thereby allowing ejection distance of the needle tube (26) to be adjusted through adjustment of mating position between the male thread portion (30d) of the ejection adjustment tube (30) and a female thread portion (29f) on an inner periphery of the elastic-member tube (22).

## Patentansprüche

1. Nadelvorrichtung für Gewebebiopsie, umfassend:
ein Ausstoßmittel, das, aus einem elastischen Element (28) gebildet, eine Ausstoßkraft erzeugt, um ein Nadelrohr (26) auszustoßen;
ein Zugmittel, das das Ausstoßmittel in eine komprimierte Position zieht, um das Nadelrohr (26) auszustoßen;
ein Bedienmittel, das das Ausstoßmittel in einer komprimierten Position hält, in die das Ausstoßmittel von dem Zugmittel gezogen wird, und das Ausstoßmittel aus der komprimierten Position freigibt und dadurch das Nadelrohr (26) ausstößt;
ein Schutzmittel, das das Bedienmittel, welches die komprimierte Position des von dem Zugmittel gezogenen Ausstoßmittels hält, blockiert; und
ein Bewegungsmittel, das das Schutzmittel aus einer Arbeitsposition, in der das Bedienmittel aktiviert ist, in eine Nichtarbeitsposition bewegt, in der das Bedienmittel entsprechend dem Ziehen des Ausstoßmittels in die komprimierte Position durch das Zugmittel blockiert ist,
**dadurch gekennzeichnet, dass**
das Bewegungsmittel einen Anschlagabschnitt (23c), der sich von einer Schutzabdeckung (23) erstreckt, bei der es sich um das direkt angrenzende Schutzmittel handelt, und einen Anschlagstreifen (29b) umfasst, der an dem Zugmittel installiert ist, das an den Anschlagabschnitt grenzt; und
der Anschlagabschnitt (23c) oder der Anschlagstreifen (29b) ein elastisches Element ist.

2. Nadelvorrichtung für Gewebebiopsie nach Anspruch 1, wobei die Schutzabdeckung (23), bei der es sich um das Schutzmittel handelt, eine Funktion zum Vermeiden von Fehlbedienungen des Bedienmittels, aufweist, wobei sie bezogen auf das Bedienmittel verschiebbar platziert ist.

3. Nadelvorrichtung für Gewebebiopsie nach Anspruch 1, wobei:
der Anschlagabschnitt (23c) ein elastisches Element ist; und
nachdem das Zugmittel gezogen wurde, der Anschlagstreifen (29b) an den Anschlagabschnitt (23c) anschlägt und die Schutzabdeckung (23) in die Nichtarbeitsposition des Bedienmittels bewegt wird, wenn das Zugmittel weitergezogen wird, wobei der Anschlagabschnitt (23c) elastisch verformt, das Zugmittel von der Schutzabdeckung (23) getrennt und die Schutzabdeckung (23) in der Nichtarbeitsposition des Bedienmittels belassen wird.

4. Nadelvorrichtung für Gewebebiopsie nach Anspruch 1, wobei:
der Anschlagstreifen (29b) ein elastisches Element ist; und
wenn das Zugmittel gezogen wird, der Anschlagstreifen (29b) an den Anschlagabschnitt (23c) anschlägt und die Schutzabdeckung (23) in die Nichtarbeitsposition des Bedienmittels bewegt wird, wobei die Schutzabdeckung (23) in der Nichtarbeitsposition des Bedienmittels platziert wird, während der Anschlagabschnitt (23c) und der Anschlagstreifen (29b) aneinander anliegen, und wenn die Schutzabdeckung (23) anschließend in die Arbeitsposition bewegt wird, der Anschlagstreifen (29b) elastisch verformt und das Zugmittel von der Schutzabdeckung (23) getrennt wird.

5. Nadelvorrichtung für Gewebebiopsie nach einem der Ansprüche 1 bis 4, wobei die Arbeitsposition, in der das Bedienmittel von dem Schutzmittel aktiviert wird, eine Position ist, in der das Schutzmittel eine Bedienung des Bedienmittels zulässt, und die Nichtarbeitsposition, in der das Bedienmittel von dem Schutzmittel blockiert ist, eine Position ist, in der das Schutzmittel die Bedienung des Bedienmittels verhindert.

6. Nadelvorrichtung für Gewebebiopsie nach einem der Ansprüche 1 bis 5, wobei das Zugmittel einen Zugknopf (20), ein elastisches Rohrelement (22), das locker an das elastische Element (28) angebaut ist, bei dem es sich um das Ausstoßmittel handelt, und ein Zugrohr (29), das in das elastische Rohrelement (22) einpasst ist, ein Ende des elastischen Elements (28) zurückhält und von dem Zugknopf (20) gegen eine elastische Kraft des elastischen Elements (28) gezogen wird, umfasst.

7. Nadelvorrichtung für Gewebebiopsie nach Anspruch 6, wobei ein Paar gegenüberliegende rechtwinklige Fenster (29d) in den Flanken des Zugrohrs (29) des Zugmittels gebildet sind, die sich in eine Längsrichtung erstrecken, und ein Ausstoßeinstellrohr (30) ausfahrbar/zurückfahrbar in der Innenperipherie des Zugrohrs (29) installiert und mit einem Außengewindeabschnitt (30d) ausgestattet ist, der durch das rechtwinklige Fenster (29d) freiliegt und dadurch erlaubt, dass der Ausstoßabstand des Nadelrohrs (26), der durch Einstellung der Passposition zwischen dem Außengewindeabschnitt (30d) des Ausstoßeinstellrohrs (30) und einem Innengewindeabschnitt (29f) auf einer Innenperipherie des Rohrs (22), bei dem es sich um ein elastisches Element handelt, einzustellen ist.

## Revendications

1. Dispositif à aiguille pour biopsie tissulaire comprenant :
des moyens d'éjection qui, étant formés d'un élément élastique (28), génèrent une force d'éjection pour éjecter un tube d'aiguille (26) ;
des moyens de traction qui tirent les moyens d'éjection jusqu'à une position comprimée pour éjecter le tube d'aiguille (26) ;
des moyens d'actionnement qui maintiennent les moyens d'éjection dans une position comprimée dans laquelle les moyens d'éjection sont tirés par les moyens de traction et libèrent les moyens d'éjection de la position comprimée, éjectant ainsi le tube d'aiguille (26) ;
des moyens de protection qui désactivent les moyens d'actionnement maintenant la position comprimée des moyens d'éjection tirés par les moyens de traction ; et
des moyens de déplacement qui déplacent les moyens de protection d'une position de travail, dans laquelle les moyens d'actionnement sont activés, à une position de non-travail, dans laquelle les moyens d'actionnement sont désactivés, en fonction de la traction des moyens d'éjection jusqu'à la position comprimée par les moyens de traction,
**caractérisé par le fait que**
les moyens de déplacement comprennent une partie de butée (23c) s'étendant à partir d'un couvercle de protection (23) qui est les moyens de protection directement en butée, et une bande de butée (29b), installée sur les moyens de traction, qui s'appuie sur la partie de butée ; et
l'une parmi la partie de butée (23c) et la bande de butée (29b) est un élément élastique.

2. Dispositif à aiguille pour biopsie tissulaire selon la revendication 1, dans lequel le couvercle de protection (23), qui est les moyens de protection, a une fonction d'empêcher des dysfonctionnements des moyens d'actionnement, en étant placé de manière coulissante par rapport aux moyens d'actionnement.

3. Dispositif à aiguille pour biopsie tissulaire selon la revendication 1, dans lequel :
la partie de butée (23c) est un élément élastique ; et
après que les moyens de traction sont tirés, mettant la bande de butée (29b) en butée contre la partie de butée (23c) et déplaçant le couvercle de protection (23) à la position de non-travail des moyens d'actionnement, lorsque les moyens de traction sont davantage tirés, la partie de butée (23c) est déformée élastiquement, séparant les moyens de traction vis-à-vis du couvercle de protection (23) et laissant le couvercle de protection (23) dans la position de non-travail des moyens d'actionnement.

4. Dispositif à aiguille pour biopsie tissulaire selon la revendication 1, dans lequel :
la bande de butée (29b) est un élément élastique ; et
lorsque les moyens de traction sont tirés, mettant la bande de butée (29b) en butée contre la partie de butée (23c) et déplaçant le couvercle de protection (23) à la position de non-travail des moyens d'actionnement, le couvercle de protection (23) est placé dans la position de non-travail des moyens d'actionnement avec la partie de butée (23c) et la bande de butée (29b) en butée l'une contre l'autre et, lorsque le couvercle de protection (23) est déplacé ultérieurement à la position de travail, la bande de butée (29b) est déformée élastiquement, séparant les moyens de traction vis-à-vis du couvercle de protection (23).

5. Dispositif à aiguille pour biopsie tissulaire selon l'une quelconque des revendications 1 à 4, dans lequel la position de travail dans laquelle les moyens d'actionnement sont activés par les moyens de protection est une position dans laquelle les moyens de protection permettent le fonctionnement des moyens d'actionnement, et la position de non-travail dans laquelle les moyens d'actionnement sont désactivés par les moyens de protection est une position dans laquelle les moyens de protection empêchent le fonctionnement des moyens d'actionnement.

6. Dispositif à aiguille pour biopsie tissulaire selon l'une quelconque des revendications 1 à 5, dans lequel les moyens de traction comprennent un bouton de traction (20), un tube d'élément élastique (22) monté de manière lâche avec l'élément élastique (28) qui est les moyens d'éjection, et un tube de traction (29) qui est monté dans le tube d'élément élastique (22), limitant une extrémité de l'élément élastique (28), et est tiré par le bouton de traction (20) à rencontre d'une force élastique de l'élément élastique (28).

7. Dispositif à aiguille pour biopsie tissulaire selon la revendication 6, dans lequel une paire de fenêtres rectangulaires opposées (29d) sont formées sur les côtés du tube de traction (29) des moyens de traction, s'étendant dans une direction longitudinale, et un tube d'ajustement d'éjection (30) est installé de manière à pouvoir avancer/reculer dans la périphérie interne du tube de traction (29), étant équipé d'une partie filetage mâle (30d) exposée à travers les fenêtres rectangulaires (29d) et permettant ainsi d'ajuster une distance d'éjection du tube d'aiguille (26) par ajustement d'une position de correspondance entre la partie filetage mâle (30d) du tube d'ajustement d'éjection (30) et une partie filetage femelle (29f) sur une périphérie interne du tube d'élément élastique (22).
